(19) Europäisches Patentamt
European Patent Office
Office européen des brevets

(11) **EP 3 248 542 A1**

(12) **EUROPEAN PATENT APPLICATION**

(43) Date of publication:
**29.11.2017 Bulletin 2017/48**

(51) Int Cl.:
*A61B 5/0456* (2006.01)   *A61B 5/046* (2006.01)
*A61B 5/0472* (2006.01)   *A61B 5/0464* (2006.01)
*A61B 5/00* (2006.01)   *A61B 5/04* (2006.01)
*A61B 5/024* (2006.01)   *A61B 5/0408* (2006.01)

(21) Application number: **17000627.4**

(22) Date of filing: **12.04.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **27.05.2016 PL 41734516**

(71) Applicant: **Comarch Healthcare
Spólka Akcyjna
31-864 Kraków (PL)**

(72) Inventors:
• **Rzepka, Dominik**
  **31-864 Kraków (PL)**
• **Pólchlopek, Wojciech**
  **31-864 Kraków (PL)**
• **Wisniewski, Marcin**
  **31-864 Kraków (PL)**
• **Kowal, Jakub**
  **37-200 Kraków (PL)**
• **Garus, Jan**
  **31-864 Kraków (PL)**

(54) **METHOD FOR AUTOMATIC DETECTION OF ATRIAL FIBRILLATION AND FLUTTER**

(57) The invention relates to a method for automatic detection of atrial fibrillation and flutter. The solution is based on ECG analysis involving QRS complex detection and atrial fibrillation and flutter interval detection. Detection of atrial fibrillation and flutter detection requires the extraction of characteristics and classification of rr signals, which includes at least one analysis of local characteristics of the signals. The QRS detection and local rr signal characteristic analysis are performed by classifiers generated during machine learning.

Fig. 2

## Description

The subject of the invention is a method for automatic detection of atrial fibrillation and flutter, especially when silent. The invention is designed for use in telemedical solutions.

Atrial fibrillation and flutter is the most common type of arrhythmia. According to published data, it is estimated that atrial fibrillation occurs with 1-2% of the general population. The frequency of occurrence greatly coincides with age. For patients below 55, only 0.1% of women and 0.2% of men is diagnosed with atrial fibrillation. For persons between 70 and 80 years of age, fibrillation occurs in 5% of men and 3.5% of women. In patients above 80 years of age, atrial fibrillation occurs in 10% of the male population. Prognoses indicate that the frequency of occurrence of this particular arrhythmia will double within the next 20-30 years, mainly due to an extended life expectancy in developed countries. Due to the abovementioned reasons, we can assume that heart arrhythmia can be considered one of the epidemics of the 21$^{st}$ century. A noticeable increase in detected heart problems is the effect of both the population's lifestyle and developments in advanced diagnostics.

The clinical course of atrial fibrillation and flutter may vary greatly, from a silent form (patient does not feel the arrhythmia) to a complex form involving multiple detrimental signals and syndromes. Among the latter, the most significant one is a low tolerance for physical exertion, caused by the atria ceasing to contract. This lowers the heart beat value by about 20%, which is particularly important for persons with heart disease or decreased contractility. A rapid and irregular transfer of the arrhythmia to the heart's ventricles can cause and/or increase damage to the left ventricle. A deterioration in patient status is often caused mainly by detectable palpitations, which are often caused by depression. However, the most harmful consequence of atrial fibrillation is an ischaemic stroke. Impairment of the atrial contractility, and therefore impairment of blood flow through the atria, can cause clotting, especially in the left atrium. This greatly increases the risk of embolic complications, including an ischaemic stroke. Ischaemic strokes occurring during atrial fibrillation have a higher mortality rate and a higher likelihood of potential patient disability when compared to patients with ischaemic strokes with different aetiology. The risk of occurrence of an ischaemic stroke in patients with atrial fibrillation is up to six times greater than in patients without arrhythmias, regardless of whether the fibrillation is chronic or paroxysmal, and symptomatic or asymptomatic. It is assumed that even a brief (5-6 minutes) asymptomatic atrial fibrillation can cause a 2.8 times greater risk of ischaemic stroke with a 2.5 times greater risk of death.

In the patent application WO2011061606 is described a solution involving the method and system for the automatic detection of atrial fibrillation. The solution is based on the analysis of the ECG signal morphology. According to the invention, detected atrial beats are used to conduct an R-R analysis. The system creates classes of R-R intervals and estimates an irregularity (deviation) index for each class. The grand mean for R-R interval deviation for all analysed atrial beats is calculated through weighted means for the irregularity factor for all classes, with the weight values equal to class sizes.

From the US patent US9307920 is known a method and an apparatus for automatic classification of arrhythmias together with an evaluation of reliability. According to the invention, the classifying system receives data about the heart from an implanted medical device. Next, the system conducts an automatic evaluation of each arrhythmia-related episode indicated by the received data. The system generates data related to and illustrating information about the episode. Data about the episode contains at least a classification of the episode resulting from the automatic episode evaluation, as well as a level of reliability of the episode's classification. In the embodiment, data on the episode can contain key characteristics, simplifying the automatic evaluation of the episode.

Another US patent US57174205 discloses system and method for diagnosing ECG signals. According to the invention, the method involves receiving an electrocardiographic signal and comparing it to signal templates of known syndromes. The solution utilises a library of defined signal samples. Distance measurements indicating a similarity of the analysed signal to previously measured signal examples are generated as a sequence of vectors. Sequences of vectors are the entry value for a classifier, which establishes the existence of template signals indicating cardiological syndromes.

The objective of the invention is to obtain a solution for detection of atrial fibrillation and flutter, which will allow an automatic detection of atrial fibrillation and flutter by the system based on received ECG signal, without the need for signal analysis by medical personnel. The solution must detect atrial fibrillation and flutter in early phases of the phenomenon. The invention will be a telemedical solution.

The invention relates to the method for automatic detection of atrial fibrillation and flutter intervals based on ECG analysis involving the detection of QRS complexes and atrial fibrillation and flutter interval detection. The invention characterised in that the detection of atrial fibrillation and flutter detection requires the extraction of characteristics and classification of *rr* signals, which includes at least one analysis of local characteristics of the signals. The QRS detection and local *rr* signal characteristic analysis are performed by classifiers generated during machine learning.

Preferably, the local *rr* signal characteristics analysis consists of the analysis of irregularities and/or bigeminy and trigeminy analysis and/or histogram analysis and/or extrema density analysis and/or analysis of hidden Markov models. The detection of atrial fibrillation and flutter intervals requires an initial rr signal processing and final processing of signals obtained by characteristic extraction and *r* signal classification. *R* signal samples are subsequent R wave locations, while *rr* signal is the sequence of distances between subsequent R waves. The detection of QRS complexes is performed on a filtered and nonlinearly transformed signal, whereas this detection requires filtering, linear and nonlinear transformation, searching for extrema, detecting false R waves and analysis of hypothetical R waves. The machine learning is based on decision trees. At least two decision trees are used simultaneously.

The main benefit of the invention is the solution which allows the automatic detection of atrial fibrillation and flutter by the system, without the need for ECG signal analysis by medical personnel. Thanks to this, examinations aimed at detecting atrial fibrillation and flutter can be performed simultaneously in a larger population. Additionally, automatization decreases the cost of examinations aimed at detecting abnormalities of atrial function. Another benefit of the invention is the fact that the system in which the atrial fibrillation and flutter detection method has been implemented is a telemedical solution. Patients can perform ECG measurements in a constant, extended manner, during their everyday activities.

The invention is presented in the embodiments on drawings where:

Fig. 1 shows a general scheme of the ECG monitoring system used for atrial flutter and fibrillation detection;
Fig. 2 shows a block diagram of the method for atrial flutter and fibrillation detection;
Fig. 3 shows a block diagram of QRS complex detection;
Fig. 4 shows a block diagram of filtration and nonlinear transformation;

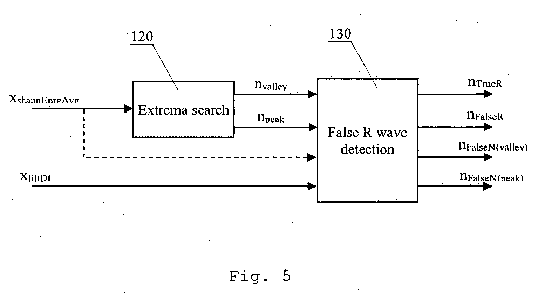

Fig. 5 shows a block diagram of extrema search and false R wave detection;
Fig. 6 shows a decision block for false R wave detection;

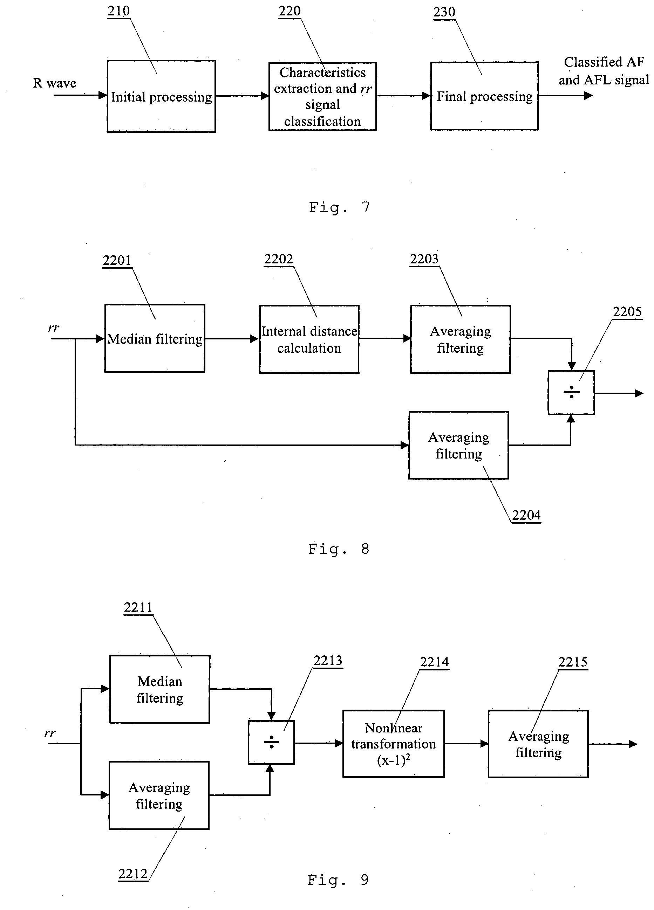

Fig. 7 shows a block diagram for AF and AFL interval detection
Fig. 8 shows a block diagram for irregularities analysis;
Fig. 9 shows a block diagram for bigeminy and trigeminy analysis;

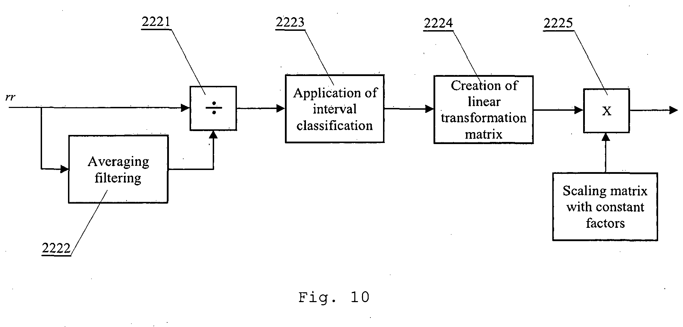

Fig. 10 shows a block diagram for hidden Markov model analysis.

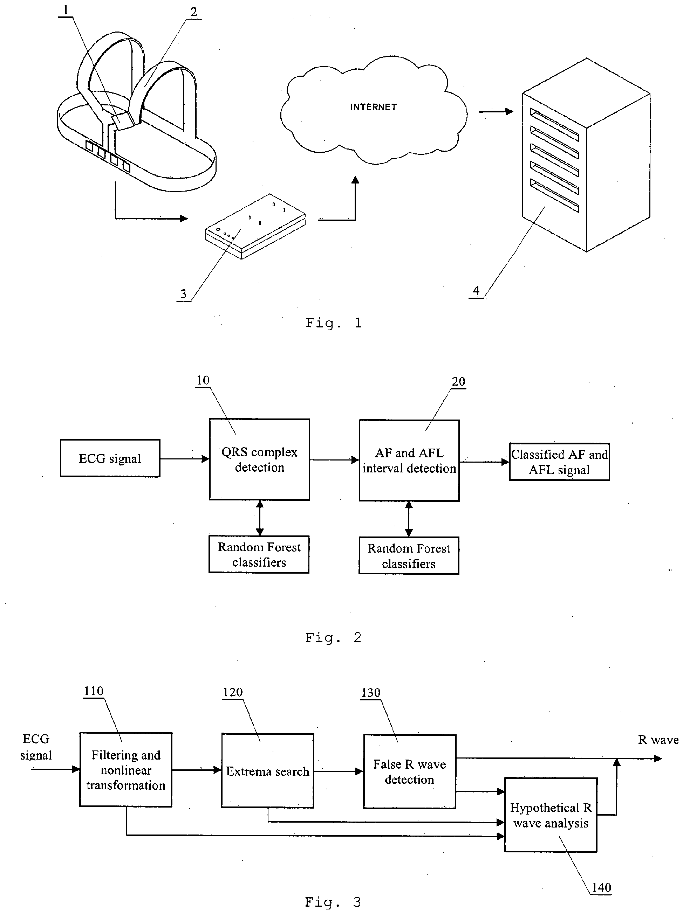

The method for automatic detection of atrial fibrillation and flutter is designed for implementation in a proprietary ECG monitoring system. Such a system, shown on fig. 1, consists of the recorder 1, garment 2 designed specifically for ECG examinations, to which electrodes have been attached, and a docking station 3. To conduct an ECG examination, the patient will put on the garment 2 and attach the recorder 1, which will record signals representing the electrical activity of the heart collected by the electrodes. Once the data has been collected, the patient will detach the recorder 1 from the vest 2 and connect it to the docking station 3, which will download the data from the recorder 1 and send them out for analysis. The docking station 3 will send the data to a server 4, where it will be processed, interpreted and shared with authorized persons. The docking station 3 sends the data received from the recorder 1 to the server 4 via the Internet. In another possible embodiment, the data could be sent using a GSM network. Thanks to remote data analysis, the ECG monitoring system is a telemedical solution. In the embodiment, the atrial flutter and fibrillation detection method described in the invention is carried out on a server 4.

According to the invention, the ECG signal collected by the electrodes, registered by the recorder 1 and sent to the server 4, undergoes analysis which first consists of QRS complex detection 10. Next, the obtained results undergo a flutter and fibrillation interval detection 20. For objective analysis, a library of algorithms for digital ECG signal processing is used. The library consists of a QRS complex detection module and an atrial fibrillation and flutter interval detection module.

As per fig. 3, showing a block diagram of QRS complex detection 10, the input ECG signal first undergoes filtering and nonlinear transformation 110. Next an extrema search 120, a false R wave detection 130 and, depending on the obtained signals, a hypothetical R wave analysis 140 are performed. Filtration and nonlinear transformation 110 produces $X_{shannEnrgAvg}$, $X_{magn}$, $X_{dtMagn}$ and $X_{filtDt}$ signals. Then extrema search 120, and the false R wave detection 130. When an extrema search 120 and false R wave detection 130 results only in marked extrema which indicate the alleged presence of R wave ($n_{peakClass}$ = *TrueR*), and/or in marked extrema indicate the alleged presence of R wave ($n_{peakClass}$ = *FalseR*), then no analysis of hypothetical R waves 140 is conducted. When, false R wave detection produces marked extrema which may have resulted from an R wave presence then further analysis of hypothetical R waves is necessary ($n_{peakClass}$ = *FalseN*) .

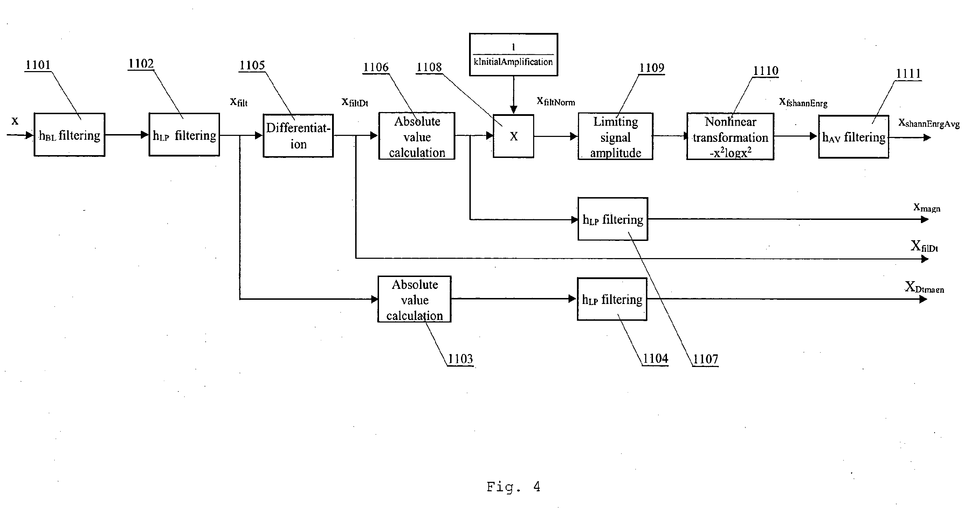

Filtering and nonlinear transformation 110 of the ECG signal have been shown in a block diagram on fig. 4. According to the invention, the input signal x is first filtered by a $h_{BL}$ filter 1101, then by a $h_{LP}$ filter 1102. The $h_{BL}$ filter is a sum of inputs from two filters: the first is cascade of two averaging filters, each with the length of $N_{BP}=0.11*f_s$, the second is the input multiplied by *(-1) and delays of $N_{PL}=0.11*f_s$ samples. The $h_{LP}$ filter is a cascade of two averaging filters with the length of $N_{LP}=0.139*f_s$ . The $h_{BL}$ and $h_{LP}$ filters are realized as a cascade of two averaging filters. The $h_{BL}$ filter is a filter removing the DC component. Since averaging filters are low-pass filters, high-pass filtering is achieved by subtracting the filtering result from the original signal with appropriate delay. The absolute value is calculated 1103 from the $x_{filt}$ signal value obtained by filtering, then the result is passed through the $h_{LP}$ filter 1104. This produces the output signal $x_{dtMagn}$. The value of the $x_{filt}$ signal is also differentiated 1105 which produces signal $x_{filtDt}$. The absolute value is calculated 1106 from the obtained $X_{filtDt}$ signal value, then the result is passed through the $h_{LP}$ filter 1107. This produces the output signal $x_{Magn}$. The value obtained by calculating the absolute value of the $x_{filtDt}$ signal is also multiplied 1108 by the value $\dfrac{1}{\text{kInitial Amplification}}$ . This produces the signal $x_{filtNorm}$. Next, the signal amplitude is limited 1109 by replacing values outside of the predefined range with the value 1. The limited signal is passed through the nonlinear transformation function $-x^2\log x^2$ 1110. The obtained $x_{shannEnrg}$ signal is passed through the $h_{AV}$ filter 1111, which produces the signal $x_{shannEnrgAvg}$. The $h_{AV}$ filter is a cascade of two average filters, with the length of $N_{AV}=0.0278{*}f_s$ each.

According to the invention, resulting signals $x_{magn}$ and $x_{dtMagn}$ are the subject of hypothetical R wave analysis 140, and signals $X_{shannEnrgAvg}$ i $X_{filtDt}$ are the subject of extrema search 120 and false R wave detection 130. Signal $X_{filtDt}$ is used directly for the false R wave detection 130, while signal $X_{shannEnrgAvg}$ is subjected to extrema search 120, then false R wave detection 130, or directly to false R wave detection 130. The extrema search 120 finds extrema $n_{valley}$ and $n_{peak}$. Those extrema are then subject to false R wave detection (...). An extremum is a sample whose both neighbouring samples are larger $x[n-1]>x[n]<[n+1]$ or smaller $x[n-1]<x[n]>[n+1]$. The relations of inequality is defined as $|x[n]-x[n-1]|>\in$ and $|x[n]-x[n+1]|>\in$.

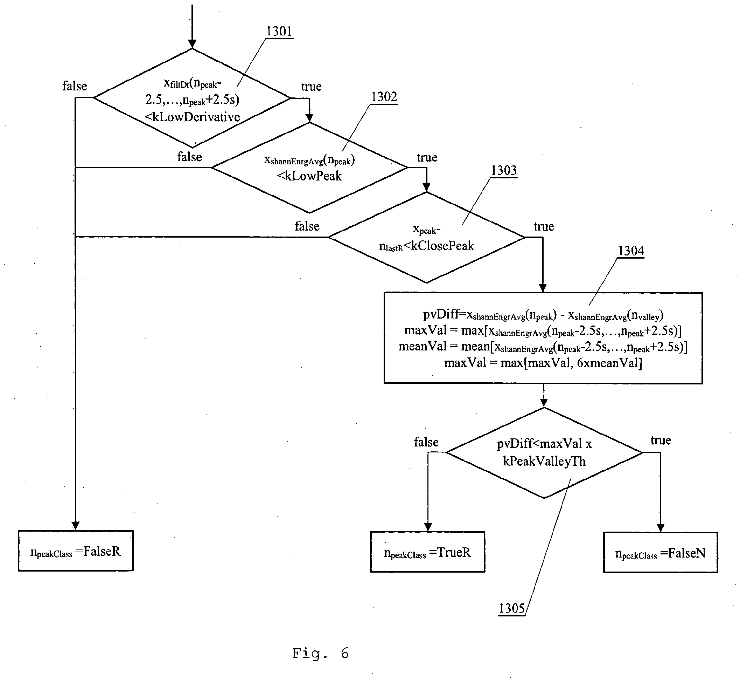

The purpose of false R wave detection (...) is marking the extrema resulting from the presence of R waves ($n_{peakClass}$ = *TrueR*), marking the extrema not resulting from R wave presence ($np_{eakClass}$ = *FalseR*), and marking extrema which may be resulting from R wave presence and therefore require further analysis ($n_{peakClass}$ = *FalseN)*. Classification of false R waves is conducted based on $x_{shannEnrgAvg}$ and $x_{filtDt}$ signals in locations indicated by $n_{peak}$ and $n_{valley}$ extrema. It is illustrated as a decision diagram in fig. 6. As per the invention, it is necessary to check whether the condition $x_{filtDt}(n_{peak}-2.5s,...,n_{peak}+2.5s)<$kLowDerivative 1301 is met. If it is not met, then the extremum does not result from the presence of R waves ($np_{eakClass}$ = *FalseR*). If, however, the condition is met, then it is necessary to check if the condition $x_{shannEnrgAvg}(n_{peak})<$kLowPeak 1302 is met. If it is not met, then we assume that extremum does not result from the presence of R waves ($n_{peakClass}$ = *FalseR*). If, however, it is met, then it is necessary to check the condition $n_{peak}-n_{LastR}<$kClosePeak 1303. If this condition is not met either, then we assume that the extremum does not result from the presence of R waves ($n_{peakClass}$ = *FalseR*). If, however, the condition is met, the following values are calculated 1304:

$$pvDiff = x_{shannEnrgAvg}\left(n_{peak}\right)\text{-}x_{shannEnrgAvg}\left(n_{valley}\right)$$

$$maxVal = \max\left[ x_{shannEnrgAvg}\left(n_{peak}-2.5s,...,n_{peak}+2.5s\right)\right]$$

$$meanVal = \text{mean}\left[ x_{shannEnrgAvg}\left(n_{peak}-2.5s,...,n_{peak}+2.5s\right)\right]$$

$$maxVal = \max\left[maxVal,6{*}meanVal\right]$$

Next, it is checked if the condition pvDiff,maxVal*kPeakValleyTh 1305 is met. If this condition is not met, then the extremum results from the presence of R waves ($np_{eakClass}$ = *TrueR*). If, however, this condition is met, we have an extremum which may result from the presence of R waves and which requires further analysis ($np_{eakClass}$ = *FalseN*).

Further analysis consists of hypothetical R wave analysis (...). The analysis is conducted using Random Forest classifiers (decision trees) generated during machine learning. The machine learning was based on a set of about a dozen decision trees. The following characteristics are taken into consideration during hypothetical R wave analysis (...):

- distance of the $n_{falseN}$ ($np_{eakClass}$ = *FalseN*) extremum from the last $n_{trueR}$ ($np_{eakClass}$ = *TrueR)* extremum before the current extremum $n_{falseN}$ ($n_{peakClass}$ = *FalseN*);
- distance of the $n_{falseN}$ ($np_{eakClass}$ = *FalseN*) extremum from the next $n_{trueR}$ (class *TrueR*) extremum before the current extremum $n_{falseN}$ ($n_{peakClass}$ = *FalseN*);
- relation between the distance of the $n_{falseN}$ ($np_{eakClass}$ = *FalseN*) extremum from the last $n_{trueR}$ ($n_{peakClass}$ = *TrueR*) extremum, and the difference between the last two extrema $n_{trueR}$ ($n_{peakClass}$ = *TrueR);*

- relation between the distance of the $n_{falseN}$ ($np_{eakClass}$ = *FalseN)* extremum from the previous $n_{trueR}$ ($np_{eakClass}$ = *TrueR)* extremum, and the difference between the last two extrema $n_{trueR}$ ($np_{eakClass}$ = *TrueR*);

- ratio $\dfrac{pv_{diff}}{x_{shannEnrgAvg}\left(n_{lastTrueR}\right)-x_{shannEnrgAvg}\left(n_{lastValley}\right)}$ , where

  - $n_{lastTrueR}$ is the position of the last detected R wave before extremum $n_{falseN}$ ($np_{eakClass}$ = *FalseN)*
  - $n_{lastValley}$ is the position of the first minimum after extremum $n_{lastTrueR}$ ($np_{eakClass}$ = *TrueR)*
  - $pv_{diff}$ is defined as $pv_{diff} = \dfrac{x_{shannEnrgAvg}\left(n_{falseNpeak}\right)-x_{shannEnrgAvg}\left(n_{falseNvalley}\right)}{\max\left[x_{shannEnrgAvg}\left(n_{falseNpeak}+n_{falseNvalley}\pm2,5s\right)\right]}$

- ratio $\dfrac{pv_{diff}}{x_{shannEnrgAvg}\left(n_{nextTrueR}\right)-x_{shannEnrgAvg}\left(n_{lastValley}\right)}$ , where

  - $n_{nextTrueR}$ is the position of the next detected R wave after extremum $n_{falseN}$ ($np_{eakClass}$ = *FalseN)*
  - $n_{nextTrueR}$ is the position of the first minimum after extremum $n_{nextTrueR\_}$ ($np_{eakClass}$ = *TrueR)*

- value of signal $x_{magn}$ (extremum $n_{falseN}$);
- value of signal $x_{magnDt}$ (extremum $n_{falseN}$);

After QRS complex detection 10, atrial fibrillation (AF) and atrial flutter (AFL) interval detection 20 is conducted. The input of the atrial fibrillation and flutter interval detection module is signal *r,* containing R wave locations. Since at the output of the QRS complex detection module some R waves occur in unrealistically small distances lesser than 100ms, the atrial fibrillation and flutter interval detection module conducts initial processing 210 which removes all such cases from the *r* sequence. During the initial processing 210, the *rr* signal, which is a sequence of distances between subsequent R waves, is also calculated. Next, the atrial fibrillation and flutter interval detection module conducts an extraction of characteristics and a classification of *rr* 220, which consists of at least one analysis of local characteristics of the *rr* signal. According to the invention, the local signal characteristics analysis consists of the analysis of irregularities and/or bigeminy and trigeminy analysis and/or histogram analysis and/or extrema density analysis and/or analysis of hidden Markov models. In the embodiment, all analyses were used. The *rr* signal classification is conducted using Random Forest classifiers (decision trees) generated during machine learning. The process of decision tree creation is based on an entropy criterion. First, several characteristics are randomly selected, out of which the one best separating classes is selected (thanks to the entropy criterion). The first degree of division (characteristic and threshold) subdivides the specimens into "left" and "right" (ones for which the inequality *characteristic < threshold* is true or not). Each subsequent division is treated like the first one, thus creating the tree structure. The leaves of the tree (indivisible end nodes) additionally contain the probability of each class occurring, which is used for establishing class membership. The atrial fibrillation/flutter detection algorithm uses 12 decision trees, whose end nodes (probabilities of each class occurring) are summed to select the class with the highest degree of probability.

In the irregularity analysis, the *rr* signal is first filtered through a median filter 2201. Internal distances are calculated in the filtered signal 2202. During internal distances calculations 2202, the number of *rr* signal samples in the window with values differing less than the assigned threshold is also calculated. The window is a signal fragment composed of a set number of successive samples, which make up the length of the window. During signal processing, the window moves by one sample, thus including successive sample groups. As per the invention, during irregularity analysis the window length is eight samples. Next, the obtained signal is filtered with an averaging filter 2203. Moreover, the *rr* signal is separately filtered using only the averaging filter 2204. Next, the relation of the calculated values is calculated 2205. During bigeminy and trigeminy analysis, the *rr* signal is filtered separately through a median filter 2211 and an averaging filter 2212. Next, the relation of the calculated values is calculated 2213. The calculated result is used as the argument of the nonlinear transformation function $(x-1)^2$ 2214 and the obtained signal is filtered using an averaging filter 2215. Histogram analysis consists of generating a histogram of *rr* signal value in a window 32 samples long. Extrema density analysis consists of establishing the relation of the number of extrema in a window 32 samples long. The Markov hidden model analysis consists of, firstly, calculating the relation 2221 of the *rr* signal to the same signal after filtering with an averaging filter 2222. To limit the number of states, an interval classification 2223 is applied with thresholds of 0.8, 1, 1.2. Secondly, a matrix of the linear transformation is created 2224 and multiplied by a scaling matrix with constant factors 2225.

According to the invention, signals obtained through characteristics extraction and *rr* signal classification 220 undergo final processing 230. The obtained signals, output atrial fibrillation and flutter intervals, often make up complexes consisting of short fragments. This type of fragmentation is often the result of improper classification. To avoid this, during final processing, a median filtration with *MedianLength* length is used for the atrial fibrillation/flutter signal supplied in a format assigning a class to every R wave, as well as interval filtration, if within the interval of assigned length *EpisodeLength* the sum of atrial fibrillation/flutter intervals is 100*duty% of the *EpisodeLength* length. The median filtration is conducted on qualified R waves. Interval filtration operates on n class signal establishing a class for all n moments of time for which ECG signal samples are available. For example, if the parameter duty=0.8 and in a time interval of a set length at least 80% of samples establishing class have the value 1 (i.e. atrial fibrillation/flutter was detected), then the entire interval will be classified as class 1.

## Claims

1. A method for automatic detection of atrial fibrillation and flutter based on ECG analysis involving the detection of QRS complexes and atrial fibrillation and flutter interval detection, **characterised in that** the detection of atrial fibrillation and flutter detection requires the extraction of characteristics and classification of *rr* signals, which includes at least one analysis of local characteristics of the signals, whereas the QRS detection and local *rr* signal characteristic analysis are performed by classifiers generated during machine learning.

2. The method according to claim 1, **characterised in that** the local *rr* signal characteristics analysis consists of the analysis of irregularities and/or bigeminy and trigeminy analysis and/or histogram analysis and/or extrema density analysis and/or analysis of hidden Markov models.

3. The method according to claim 1, **characterised in that** the detection of atrial fibrillation and flutter intervals requires an initial *rr* signal processing and final processing of signals obtained by characteristic extraction and *r* signal classification.

4. The method according to claim 1, **characterised in that** r signal samples are subsequent R wave locations, while *rr* signal is the sequence of distances between subsequent R waves.

5. The method according to claim 1, **characterised in that** the detection of QRS complexes is performed on a filtered and nonlinearly transformed signal, whereas this detection requires filtering, linear and nonlinear transformation, searching for extrema, detecting false R waves and analysis of hypothetical R waves.

6. The method according to claim 1, **characterised in that** the machine learning is based on decision trees.

7. The method according to claim 6, **characterised in that** at least two decision trees are used simultaneously.

Fig. 1

Fig. 2

Fig. 3

Fig. 4

Fig. 5

Fig. 6

1301: $X_{filtDt}(n_{peak}-2.5,...,n_{peak}+2.5s) < kLowDerivative$ — false → $n_{peakClass} = FalseR$ ; true →

1302: $X_{shannEngrAvg}(n_{peak}) < kLowPeak$ — false → $n_{peakClass} = FalseR$ ; true →

1303: $x_{peak} - n_{lastR} < kClosePeak$ — false → $n_{peakClass} = FalseR$ ; true →

1304:
$$pvDiff = X_{shannEngrAvg}(n_{peak}) - X_{shannEngrAvg}(n_{valley})$$
$$maxVal = max[X_{shannEngrAvg}(n_{peak}-2.5s,...,n_{peak}+2.5s)]$$
$$meanVal = mean[x_{shannEngrAvg}(n_{peak}-2.5s,...,n_{peak}+2.5s)]$$
$$maxVal = max[maxVal, 6 \times meanVal]$$

1305: $pvDiff < maxVal \times kPeakValleyTh$ — false → $n_{peakClass} = TrueR$ ; true → $n_{peakClass} = FalseN$

Fig. 7

Fig. 8

Fig. 9

*rr*

2221

÷

2223

Application of interval classification

2224

Creation of linear transformation matrix

2225

X

Averaging filtering

2222

Scaling matrix with constant factors

Fig. 10

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/164349 A1 (GOPALAKRISHNAN RAVI [US] ET AL) 18 June 2015 (2015-06-18) * paragraphs [0009], [0050] - [0053], [0055], [0057], [0058], [0070] * * table 2 * ----- | 1-7 | INV. A61B5/0456 A61B5/046 A61B5/0472 A61B5/0464 A61B5/00 A61B5/04 A61B5/024 |
| X | WO 2016/077786 A1 (ZOLL MEDICAL CORP [US]; WENG BINWEI [US]; JOHNSON GUY R [US]) 19 May 2016 (2016-05-19) * page 73, line 5 - line 28 * * page 58, lines 5-11 * * figures 1-3, 8 * ----- | 1-7 | ADD. A61B5/0408 |
| A | WO 2008/007236 A2 (KONINKL PHILIPS ELECTRONICS NV [NL]; PHILIPS INTELLECTUAL PROPERTY [DE] 17 January 2008 (2008-01-17) * the whole document * ----- | 1-7 | |
| A | US 2006/276716 A1 (HEALEY JENNIFER [US] ET AL) 7 December 2006 (2006-12-07) * the whole document * ----- | 1-7 | |
| A | WO 2006/091636 A2 (DIGITAL INTELLIGENCE L L C [US]; RICCI CARLOS A [US]; KOVTUN VLADIMIR) 31 August 2006 (2006-08-31) * the whole document * ----- | 1-7 | TECHNICAL FIELDS SEARCHED (IPC)  A61B |
| A | KOEHLER B-U ET AL: "THE PRINCIPLES OF SOFTWARE QRS DETECTION REVIEWING AND COMPARING ALGORITHMS FOR DETECTING THIS IMPORTANT ECG WAVEFORM", IEEE ENGINEERING IN MEDICINE AND BIOLOGY MAGAZINE, IEEE SERVICE CENTER, PISACATAWAY, NJ, US, vol. 21, no. 1, 1 January 2002 (2002-01-01), pages 42-57, XP001184465, ISSN: 0739-5175, DOI: 10.1109/51.993193 * the whole document * ----- | 1-7 | |

The present search report has been drawn up for all claims

| Place of search | Date of completion of the search | Examiner |
|---|---|---|
| The Hague | 10 October 2017 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 00 0627

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2017

| Patent document cited in search report | | | Publication date | Patent family member(s) | | | Publication date |
|---|---|---|---|---|---|---|---|
| US | 2015164349 | A1 | 18-06-2015 | EP | 3079571 | A1 | 19-10-2016 |
| | | | | US | 2015164349 | A1 | 18-06-2015 |
| | | | | US | 2015265164 | A1 | 24-09-2015 |
| | | | | US | 2017238814 | A1 | 24-08-2017 |
| | | | | WO | 2015089484 | A1 | 18-06-2015 |
| WO | 2016077786 | A1 | 19-05-2016 | EP | 3204881 | A1 | 16-08-2017 |
| | | | | US | 2016135706 | A1 | 19-05-2016 |
| | | | | WO | 2016077786 | A1 | 19-05-2016 |
| WO | 2008007236 | A2 | 17-01-2008 | NONE | | | |
| US | 2006276716 | A1 | 07-12-2006 | NONE | | | |
| WO | 2006091636 | A2 | 31-08-2006 | US | 2006200034 | A1 | 07-09-2006 |
| | | | | US | 2006200035 | A1 | 07-09-2006 |
| | | | | US | 2010195770 | A1 | 05-08-2010 |
| | | | | US | 2011313760 | A1 | 22-12-2011 |
| | | | | US | 2013282339 | A1 | 24-10-2013 |
| | | | | US | 2016087603 | A1 | 24-03-2016 |
| | | | | WO | 2006091636 | A2 | 31-08-2006 |

EPO FORM P0459

For more details about this annex : see Official Journal of the European Patent Office, No. 12/82

**REFERENCES CITED IN THE DESCRIPTION**

**Patent documents cited in the description**

- WO 2011061606 A
- US 9307920 B
- US 57174205 B